Europäisches Patentamt

European Patent Office

Office européen des brevets

(11) Veröffentlichungsnummer: **0 330 047 B1**

(12) **EUROPÄISCHE PATENTSCHRIFT**

(45) Veröffentlichungstag der Patentschrift: **23.09.92**

(51) Int. Cl.⁵: **A61L 2/18**

(21) Anmeldenummer: **89102469.7**

(22) Anmeldetag: **14.02.89**

(54) **Verfahren zur Sterilisation von Blut, Plasma, Blut- und Plasmaderivaten, Zellsuspensionen oder dgl.**

(30) Priorität: **22.02.88 DE 3805459**

(43) Veröffentlichungstag der Anmeldung:
**30.08.89 Patentblatt 89/35**

(45) Bekanntmachung des Hinweises auf die
Patenterteilung:
**23.09.92 Patentblatt 92/39**

(84) Benannte Vertragsstaaten:
**AT BE CH DE ES FR GB GR IT LI LU NL SE**

(56) Entgegenhaltungen:

CHEMICAL ABSTRACTS, Band 103, Nr. 7, 19.
August 1985, Seite 315, Zusammenfassung
Nr. 51128a, Columbus, Ohio, US; A. MURATA
et al.: "A new biological activity in vitro of
fat-soluble vitamins and related substances:
phage-inactivating effect of vitamins E and
K, and coenzyme O", & AGRIC. BIOL. CHEM.
1985, 49(6), 1903-4

CHEMICAL ABSTRACTS, Band 82, Nr. 9, 3.
März 1975, Seite 4, Zusammenfassung Nr.
51222z, Columbus, Ohio, US; A. MURATA:
"Inactivation of viruses by vitamin C. Mechanism of the inactivation", & VITAMINS 1974,
48(10-11), 507-11

(73) Patentinhaber: **BIOTEST PHARMA GMBH**
**Landsteiner Strasse 5**
**W-6072 Dreieich(DE)**

(72) Erfinder: **Dichtelmüller, Herbert, Dr.**
**Rossertstrasse 14**
**W-6231 Sulzbach/Ts(DE)**
Erfinder: **Stephan Wolfgang, Dr. Dipl.-Chem.**
**Philipp-Holzmann-Strasse 84**
**W-6072 Dreieich(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**W-6230 Frankfurt am Main 80(DE)**

## Beschreibung

Die Erfindung betrifft das in den Patentansprüchen beschriebene Verfahren zur Sterilisation von Blut, Plasma, Blut- und Plasmaderivaten, Zellsuspensionen oder dgl. mit einem physiologisch unbedenklichen Sterilisationsmittel.

Humanblut bzw. Humanplasma oder daraus hergestellte Produkte bergen potentiell das Risiko, virale Infektionen zu übertragen, insbesondere spielen humanpathogene Lipidviren, wie HIV, HNANBV und HBV, eine besondere Rolle. Aus diesem Grunde müssen Präparate solcher Herkunft einem Sterilisationsschritt, d.h. einer Virusinaktivierung, unterzogen werden.

Gerinnungspräparate, wie F VIII oder F IX Konzentrate, können mittels Hitze sterilisiert werden (EP 0 037 078), benötigen jedoch beträchtliche Mengen an Stabilisatoren, um eine solche Behandlung zu überstehen. Bei anderen Verfahren wird das Präparat im lyophilisierten Zustand erhitzt, wobei zusätzlich organische Lösungsmittel, Druck oder Dampf in Kombination verwendet werden.

Ebenso werden chemische Agenzien, wie eine Kombination von Lipidlösungsmittel und Detergentien oder $\beta$-Propiolacton in Kombination mit UV-Bestrahlung angewandt (EP 0 014 333).

Alle diese Verfahren weisen unterschiedliche Vorteile auf, jedoch auch Nachteile, welche die Anwendung oder die Wirksamkeit beeinträchtigen. Die zur Hitzesterilisation in Lösung zugesetzten Substanzen müssen im Endprodukt bei Gerinnungsfaktoren wieder entfernt werden. Bei Erhitzen im lyophilisierten Zustand bestimmt der schlecht standardisierbare Gehalt an Restfeuchte die Effektivität des Verfahrens, ausserdem können zahlreiche Blutbestandteile nicht lyophilisiert werden. $\beta$-Propiolacton/UV ist für die Sterilisation von F VIII nur unter Ausbeuteverlusten geeignet, Lösungsmittel und Detergentien müssen aus dem biologischen Material durch aufwendige Prozesse wieder entfernt werden.

Bisher nicht möglich ist die Sterilisation von Zellsuspensionen. Z.B. eignen sich für Erythrozytensuspensionen Hitzesterilisationsverfahren nicht, da die Zellen zerstört werden. Ebenso bedingen chemische Verfahren (Detergentien, Lösungsmittel oder $\beta$-Propiolacton) eine vollständige oder partielle Hämolyse der Zellen. Auch Suspensionen weisser Blutzellen sind durch diese Verfahren nicht sterilisierbar.

Zusammenfassend gilt, dass bei allen beschriebenen Sterilisationsverfahren unphysiologische Bedingungen und Substanzen angewandt werden.

Der Erfindung liegt die Aufgabe zugrunde, ein Sterilisationsverfahren (Virusinaktivierung) bereitzustellen, welches unter physiologischen Bedingungen und mit physiologischen Substanzen arbeitet, welches sowohl für die Sterilisation von Blut/Plasma oder deren Derivate als auch für Zellsuspensionen geeignet ist und bei dem als sterilisierendes Agens eine physiologisch gut verträgliche Substanz verwendet wird, die im zu sterilisierenden biologischen Material verbleiben kann.

Erfindungsgemäss wurde diese Aufgabe dadurch gelöst, dass als sterilisierendes Agens Vitamine verwendet werden. Vitamine sind natürliche physiologische Substanzen, deren antiinfektiöse, gesundheitsfördernde Wirkung bereits bekannt ist. Vitamine müssen zudem nicht aus dem zu sterilisierenden biologischen Material entfernt werden, da der menschliche Körper auf die Zufuhr von Vitaminen angewiesen ist. Bevorzugte Vitamine sind z.B. Vitamin A, der Vitamin B-Komplex B1, B6, B12, Vitamin C und Vitamin E. Bevorzugte Salze hiervon sind die Acetate, wie z.B. Vitamin A- und Vitamin E-Acetat.

Die Sterilisation erfolgt in der Weise, dass man das zu sterilisierende biologische Material mit 0,01 bis 1,0 Gew.-% eines Vitamins (pro Volumen-% Sterilisat) versetzt, den pH-Wert auf 5,6 bis 8,5 einstellt und unter Rühren bei 10°C bis 50°C bis zu 24 Stunden lang inkubiert. Vorzugsweise beträgt der pH-Wert 7,15 und die Temperatur 25 bis 37°C und besonders bevorzugt 23°C bzw. 37°C. Die bevorzugte Sterilisationszeit beträgt 3 bis 4 Stunden. Falls notwendig, kann zur Verbesserung der Löslichkeit des Vitamins ein Lösungsvermittler zugesetzt werden. Ein bevorzugter Lösungsvermittler ist Tween 80.

Mit dem erfindungsgemässen Verfahren können Blut, Blutzellen, Blutplasma, Blutserum, Inhaltsstoffe von Blutzellen, wie beispielsweise Interferone, Interleukine, Hämoglobine, Konzentrate roter Blutzellen und anderer Blutinhaltsstoffe wie Granulocyten, Blättchen, Lymphozyten und dgl. sowie Plasmaderivate wie z.B. Plasma ohne Gerinnungsfaktoren, Gerinnungsfaktoren wie z.B. Faktor VIII, IX, X, XIII, Kryopräzipitat, Immunglobuline oder die Inhibitoren $\alpha_1$-Antitrypsin, Antithrombin III u.ä. sterilisiert werden.

Das sterilisierende Agens, i.e. das Vitamin oder dessen Salz, kann in dem zu sterilisierenden Material verbleiben. Man kann es auch auf einfache Weise durch Waschen, Gelfiltration, Adsorption oder Ultrafiltration wieder daraus entfernen.

In den nachfolgenden Beispielen wurden zum Nachweis der erf indungsgemässen Virusinaktivierung Sendaiviren verwendet, die sich als Marker-Viren für die humanpathogenen Lipidviren HBV, NHANBV und HIV experimentell gut bewährt haben. Ferner wurden Herpes-Viren HSV-I verwendet.

Beispiel 1

Sterilisation von Plasma mittels Vitamin C

100 ml Humanplasma wurden mit 0,2 % Vitamin C versetzt, nachdem das Plasma mit Sendaivirus kontaminiert worden war. Die Mischung wurde 4 Stunden bei 23°C und pH 7,15 inkubiert.

Eine ebenfalls mit Sendaivirus kontaminierte Vergleichsprobe wurde unter gleichen Bedingungen, jedoch ohne Vitaminzusatz, inkubiert. Die Proben wurden unmittelbar nach Inkubation bei -80°C eingefroren. Die Infektionstiter von Sendaivirus wurden auf Bruteiern bestimmt. In der Vergleichsprobe wurde ein Titer von $10^{2,7}$ infektiösen Einheiten/ml gefunden. In der mit Vitamin C versetzten Probe war kein infektiöses Virus mehr nachweisbar, entsprechend einer Inaktivierung von $\geqq 2{,}7$ $\log_{10}$.

Beispiel 2

Humanerythrocyten, welche dreimal mit 0,9%igem NaCl gewaschen worden waren, wurden mit Sendai-Virus kontaminiert (Verdünnung 1:100). Anschliessend wurde die Hälfte der kontaminierten Erythrocyten mit 0,25% Vitamin E-Acetat versetzt und die Mischung bei 37°C 3 Stunden inkubiert. Die andere kontaminierte Erythrocytenhälfte wurde ohne Vitamine inkubiert. In allen nachfolgenden Beispielen wurde die Kontrollprobe analog behandelt. Der Infektionstiter von Sendai-Virus wurde auf Bruteiern bestimmt.

In der mit Vitamin E-Acetat behandelten Probe war kein infektiöses Virus mehr nachweisbar, entsprechend einer Inaktivierung von $>4{,}7$ $\log_{10}$, während in der Vergleichsprobe ein Titer von $5 \times 10^4$ infektiöse Einheiten/ml gefunden wurde.

Diese Ergebnisse sind in nachfolgender Tabelle 1 zusammengefasst.

Tabelle I

| Inaktivierung von Sendai-Virus in Human-erythrocytenkonzentrat durch Vitamin E | | | | |
|---|---|---|---|---|
| Virus | Behandlung | Titer unbehandelte Probe | Titer Vit.E-behandelte Probe | Inaktivierung ($\log_{10}$) |
| Sendai | Vit.E-Acetat 0,25% 3h, 37°C | $5 \times 10^4$ | $< 10^0$ | $>4{,}7$ |

Beispiel 3

Humanerythrocyten wurden mit Herpes Virus (HSV-I) kontaminiert und wie in Beispiel 2 beschrieben mit Vitamin E-Acetat behandelt. Zusätzlich wurde zur besseren Löslichkeit Tween 80 (0,075%/0,01%) zugegeben.

Die Infektionstiter wurden auf Zellkulturen (Rita-Zellen) bestimmt. Die Ergebnisse sind in nachfolgender Tabelle II zusammengefasst. Wie hieraus ersichtlich ist, erfolgt mit Tween 80 allein keine Virusinaktivierung. Selbst 10fach höhere Konzentrationen als für die Kombination mit Vitamin E blieben wirkungslos.

Tabelle II

| Inaktivierung von Herpes Virus (HSV-I) in humanem Erythrocytenkonzentrat durch Vitamin E | | |
|---|---|---|
| Virus | Behandlung | Inaktivierung ($\log_{10}$) |
| Herpes | Vitamin E-Acetat + Tween 80 (0,15% + 0,037%) | 2,7 |
| | Vitamin E-Acetat + Tween 80 (0,05% + 0,01%) | 2,3 |
| | Vitamin E + Tween 80 (0,01% + 0,002%) | 1,3 |
| | Tween 80, 0,15% | 0,3 |

Beispiel 4

Humanplasma wurde mit Sendai-Virus (Verdünnung 1:50) kontaminiert. Ein Teil wurde in Gegenwart von 0,2% Vitamin A-Acetat bei 23°C 3 Stunden inkubiert. Die Infektionstiter wurden wie in Beispiel 1 beschrieben bestimmt. Die Vitamin A-Acetat-Behandlung führte zu einer Inaktivierung des Sendai-Virus von

>2,7 $\log_{10}$ (Tabelle III).

## Beispiel 5

Mit Sendai-Virus kontaminiertes Humanplasma wurde mit 0,5% Vitamin A-Acetat und 0,15% Tween 80 versetzt und wie in Beispiel 4 beschrieben inkubiert.
Die Virusinaktivierung betrug 2,0 $\log_{10}$ (Tabelle III).

Tabelle III

| Inaktivierung von Sendai-Virus durch Vitamin E-Acetat in Humanplasma | | |
|---|---|---|
| Virus | Behandlung | Inaktivierung ($\log_{10}$) |
| Sendai | Vit. A-Acetat <br> Vit.A-Acetat + Tween 80 (0,5% + 0,07%) | >2,7 <br> 2,0 |

## Beispiel 6

Humanplasma, in gleicher Weise wie in Beispiel 1 mit Sendaivirus kontaminiert, wurde mit 0,2 % Vitamin B 6 versetzt und bei 23°C 3 Stunden bei pH 7,2 inkubiert. Nach der Inkubation war kein infektiöses Sendai-Virus mehr nachweisbar, entsprechend einer Inaktivierung von $\geq 2,7$ $\log_{10}$. Die Behandlung mit 0,1% Vitamin B6 führte zu einer Inaktivierung von 1,0 $\log_{10}$ (Tabelle IV).

## Beispiel 7

Humanplasma, in gleicher Weise wie in Beispiel 1 mit Sendai-Virus kontaminiert, wurde mit 0,2 % Vitamin B 12 versetzt und wie in Beispiel 6 beschrieben vorgegangen. Nach 3 Stunden Inkubation war kein infektiöses Sendai-Virus mehr nachweisbar, entsprechend einer Inaktivierung von $\geq 2,7$ $\log_{10}$.

Tabelle IV

| Inaktivierung von Sendai-Virus in Humanplasma durch Vitamin B | | | |
|---|---|---|---|
| Virus | Vitamin | Behandlung | Inaktivierung ($\log_{10}$) |
| Sendai | B6 | 0,2%, 3 Std., 23°C | >2,7 |
| Sendai | B6 | 0,1%, 3 Std., 23°C | 1,0 |
| Sendai | B12 | 0,2%, 3 Std., 23°C | >2,7 |

## Beispiel 8

Humanerythrozyten wurden mit Herpes-Virus (HSV-I) kontaminiert und mit Vitamin A-Acetat (0,2%) zusammen mit Tween 80 (0,075%) versetzt.
Die Virusinaktivierung betrug 2,0 $\log_{10}$ (Tabelle V).

## Beispiel 9

Humanerythrozyten wurden mit Herpes-Virus (HSV-I) kontaminiert und mit Vitamin B12 versetzt. Die Inaktivierung von HSV-I betrug 1,0 $\log_{10}$ (Tabelle V).

EP 0 330 047 B1

Tabelle V

| Inaktivierung von Herpes-Virus (HSV-I) in Humanerythrocyten durch Vitamin A und B12 | | | |
|---|---|---|---|
| Virus | Vitamin | Behandlung | Inaktivierung ($\log_{10}$) |
| HSV-I | Vit.A-Acetat (0,2%)  +  Tween 80 (0,075%) | 3Std.,23°C | 2,0 |
| HSV-I | Vit.B12 (0,2%) | 3Std.,23°C | 1,0 |

**Patentansprüche**

1. Verfahren zur Sterilisation von Blut, Plasma, Blut-und Plasmaderivaten, Zellsuspensionen oder dgl., dadurch gekennzeichnet, dass als sterilisierendes Agens Vitamine und Provitamine verwendet werden.

2. Verfahren nach Anspruch 1, dadurch gekennzeichnet, dass die Vitamine oder Provitamine in Form von Salzen oder Derivaten verwendet werden.

3. Verfahren nach Anspruch 1 oder 2, dadurch gekennzeichnet, dass eine Kombination von Vitaminen verwendet wird.

4. Verfahren gemäss Anspruch 1, dadurch gekennzeichnet, dass Vitamin A verwendet wird.

5. Verfahren gemäss Anspruch 1, dadurch gekennzeichnet, dass Vitamin E verwendet wird.

6. Verfahren gemäss Anspruch 1, dadurch gekennzeichnet, dass Vitamin B oder Vitamin C verwendet wird.

7. Verfahren gemäss Anspruch 2 oder 3, dadurch gekennzeichnet, dass Vitamin A-Acetat und/oder Vitamin E-Acetat verwendet wird.

8. Verfahren gemäss Anspruch 1 oder 6, dadurch gekennzeichnet, dass Vitamin B1, B6 oder B12 verwendet wird.

9. Verfahren gemäss Anspruch 1-8, dadurch gekennzeichnet, dass zusätzlich Mittel zur Verbesserung der Löslichkeit der Vitamine in wässriger Lösung eingesetzt werden.

10. Verfahren gemäss Anspruch 9, dadurch gekennzeichnet, dass als zusätzliches Mittel Tween 80 verwendet wird.

11. Verfahren nach Anspruch 1 bis 10, dadurch gekennzeichnet, dass die Vitamine in einer Konzentration von 0,01 bis 1,0 Gew.-% pro Volumen-% Sterilisat verwendet werden.

12. Verfahren nach Anspruch 1 bis 11, dadurch gekennzeichnet, dass die Sterilisation bei einer Temperatur von 10°C bis 50°C, vorzugsweise bei 25°C bis 37°C und besonders bevorzugt bei 23°C oder 37°C durchgeführt wird.

13. Verfahren nach Anspruch 1 bis 12, dadurch gekennzeichnet, dass die Sterilisationszeit 1 bis 24 Stunden, vorzugsweise 3 bis 4 Stunden, beträgt.

14. Verfahren nach Anspruch 1-13, dadurch gekennzeichnet, dass das zu sterilisierende Blutmaterial ein Konzentrat roter Blutzellen oder ein Konzentrat anderer Blutinhaltsstoffe wie Granulocyten, Blättchen, Lymphocyten oder Blutserum ist.

15. Verfahren nach Anspruch 1 bis 13, dadurch gekennzeichnet, dass das zu sterilisierende Plasmamaterial Humanimmunoglobulin, Gerinnungsfaktoren wie Faktor VIII, IX, X, XIII etc., Plasma ohne Gerinnungsfaktoren oder Kryopräzipitat ist.

5

EP 0 330 047 B1

**16.** Verfahren nach Anspruch 15, dadurch gekennzeichnet, dass das zu sterilisierende Plasmamaterial die Inhibitoren $\alpha_1$-Antityrpsin oder Antithrombin III ist.

**17.** Verfahren nach Anspruch 1 bis 16, dadurch gekennzeichnet, dass das sterilisierende Agens durch Waschen, Gelfiltration, Adsorption oder Ultrafiltration von dem zu sterilisierenden Material entfernt wird.

**Claims**

**1.** A process for the sterilization of blood, plasma, blood and plasma derivatives, cell suspensions or the like, characterised in that the sterilizing agents used are vitamins and provitamins.

**2.** A process according to Claim 1, characterised in that the vitamins or provitamines are used in the form of salts or derivatives.

**3.** A process according to Claim 1 or 2, characterised in that a combination of vitamins is used.

**4.** A process according to Claim 1, characterised in that Vitamin A is used.

**5.** A process according to Claim 1, characterised in that Vitamin E is used.

**6.** A process according to Claim 1, characterised in that Vitamin B or Vitamin C is used.

**7.** A process according to Claim 2 or 3, characterised in that Vitamin A-acetate and/or Vitamin E-acetate is used.

**8.** A process according to Claim 1 or 6, characterised in that Vitamin B1, B6 or B12 is used.

**9.** A process according to Claims 1-8, characterised in that agents for improving the solubility of the vitamins in aqueous solution are used in addition.

**10.** A process according to Claim 9, characterised in that Tween 80 is used as additional agent.

**11.** A process according to Claims 1 to 10, characterised in that the vitamins are used at a concentration of from 0.01 to 1.0% by weight per volumes percent of sterilized product.

**12.** A process according to Claims 1 to 11, characterised in that sterilization is carried out at a temperature of from 10°C to 50°C, preferably at 25°C to 37°C and most preferably at 23°C or 37°C.

**13.** A process according to Claims 1 to 12, characterised in that the sterilization time is from 1 to 24 hours, preferably from 3 to 4 hours.

**14.** A process according to Claims 1 to 13, characterised in that the blood material to be sterilized is a concentrate of red blood cells or a concentrate of other blood components such as granulocytes, platelets, lymphocytes or blood serum.

**15.** A process according to Claims 1 to 13, characterised in that the plasma material to be sterilized is human immunoglobulin, clotting factors such as Factor VIII, IX, X, XIII, etc., plasma without clotting factors or cryoprecipitate.

**16.** A process according to Claim 15, characterised in that the plasma material to be sterilized consists of the inhibitors $\alpha_1$-antitrypsine or antithrombin

**17.** A process according to Claims 1 to 16, characterised in that the sterilizing agent is removed from the material to be sterilized by washing, gel filtration, adsorption or ultrafiltration.

**Revendications**

**1.** Procédé de stérilisation de sang, de plasma, de dérivés du sang et du plasma, de suspensions

6

EP 0 330 047 B1
# EP 0 330 047 B1

cellulaires ou analogues, caractérisé en ce qu'on utilise, comme agent stérilisant, des vitamines et des provitamines.

**2.** Procédé selon la revendication 1, caractérisé en ce que les vitamines ou provitamines sont utilisées sous forme de sels ou de dérivés.

**3.** Procédé selon la revendication 1 ou 2, caractérisé en ce qu'on utilise une combinaison de vitamines.

**4.** Procédé selon la revendication 1, caractérisé en ce qu'on utilise la vitamine A.

**5.** Procédé selon la revendication 1, caractérisé en ce qu'on utilise la vitamine E.

**6.** Procédé selon la revendication 1, caractérisé en ce qu'on utilise la vitamine B ou la vitamine C.

**7.** Procédé selon la revendication 2 ou 3, caractérisé en ce qu'on utilise l'acétate de vitamine A et/ou l'acétate de vitamine E.

**8.** Procédé selon la revendication 1 ou 6, caractérisé en ce qu'on utilise la vitamine B1, B6 ou B12.

**9.** Procédé selon les revendications 1-8, caractérisé en ce qu'on ajoute en plus des agents pour améliorer la solubilité des vitamines en solution aqueuse.

**10.** Procédé selon la revendication 9, caractérisé en ce qu'on utilise le Tween 80 comme agent additionnel.

**11.** Procédé selon les revendications 1 à 10, caractérisé en ce qu'on utilise les vitamines à une concentration de 0,01 à 1,0 % en poids par volume de produit stérilisé.

**12.** Procédé selon les revendications 1 à 11, caractérisé en ce que la stérilisation est effectuée à une température de 10°C à 50°C, de préférence de 25°C à 37°C et de façon particulièrement préférée à 23°C ou à 37°C.

**13.** Procédé selon les revendications 1 à 12, caractérisé en ce que le temps de stérilisation est de 1 à 24 heures, de préférence de 3 à 4 heures.

**14.** Procédé selon les revendications 1 à 13, caractérisé en ce que le produit sanguin à stériliser est un concentré de globules rouges ou un concentré d'autres constituants du sang tels que des granulocytes, des plaquettes, des lymphocytes ou du sérum sanguin.

**15.** Procédé selon les revendications 1 à 13, caractérisé en ce que le produit plasmatique à stériliser est une immunoglobuline humaine, des facteurs de coagulation comme les facteurs VIII, IX, X, XIII etc., du plasma exempt de facteurs de coagulation ou un cryoprécipité.

**16.** Procédé selon la revendication 15, caractérisé en ce que le produit plasmatique à stériliser est l'inhibiteur $\alpha_1$-antitrypsine ou l'inhibiteur antithrombine III.

**17.** Procédé selon les revendications 1 à 16, caractérisé en ce que l'agent stérilisant est éliminé du produit à stériliser par lavage, filtration sur gel, adsorption ou ultrafiltration.